# EUROPEAN PATENT APPLICATION

(11) **EP 2 851 082 A1**
(43) Date of publication of application: **25.03.2015**
(21) Application number: 13784252.2
(22) Date of filing: 30.04.2013
(51) Int. Cl.: A61K 38/00, A23K 1/16, A23L 1/30, A23L 1/305, A61P 19/02

(54) **CARTILAGE REGENERATION-PROMOTING AGENT**

(30) Priority: 02.05.2012 JP 2012105509
(71) Applicant: Megmilk Snow Brand Co. Ltd., Sapporo-shi, Hokkaido 065-0043 (JP)
(72) Inventor: TAKANO, Yoshihiko, Sapporo-shi Hokkaido 065-0043 (JP); NARA, Takayuki, Sapporo-shi Hokkaido 065-0043 (JP); KATO, Ken, Sapporo-shi Hokkaido 065-0043 (JP); MORITA, Yoshikazu, Sapporo-shi Hokkaido 065-0043 (JP)
(74) Representative: Høiberg A/S
(86) International application number: PCT/JP2013/062525
(87) International publication number: WO 2013/164992

(57) **Abstract**

It is an object of the present invention to provide a chondrogenesis promoter that is safe and exhibits a chondrogenesis-promoting effect by daily ingestion. It is also an object of the invention to provide a proteoglycan synthesis promoter that exhibits a proteoglycan synthesis-promoting effect by ingestion. Provided are a chondrogenesis promoter and a proteoglycan synthesis promoter each containing a milk-derived basic protein fraction as an active ingredient. Synthesis of proteoglycan and formation of a cartilage can be promoted by oral ingestion of the milk-derived basic protein fraction. A hydrolysate of the milk-derived basic protein fraction also has a similar chondrogenesis-promoting effect.

## Description

### TECHNICAL FIELD

The present invention relates to a chondrogenesis promoter containing a milk-derived basic protein fraction as an active ingredient and promoting chondrogenesis, and relates to chondrogenesis-promoting food or feed containing the chondrogenesis promoter.

### BACKGROUND ART

Cartilage is present in joints, nasal parts, auricular regions, and other parts, among which articular cartilage has a role of helping individual motor function.

According to the results of ROAD project research in 2009, the number of Japanese patients suffering from osteoarthritis (OA) of the knee is 25000000, and that of the lumbar spine is 38000000. The breakdowns of male and female patients are 8600000 male patients versus 16700000 female patients in OA of the knee and 18900000 male patients versus 19000000 female patients in OA of the lumbar spine. These numbers are increasing yearly. OA depresses the activity of daily living (ADL) and the quality of life (QOL) of the elderly as in osteoporosis, and radical treatment thereof is desired to be established.

The articular cartilage tissue is composed of collagen, hyaluronic acid, and proteoglycan generated by chondrocytes and retains a large amount of moisture by the presence of proteoglycan and hyaluronic acid between collagen fibers forming a network structure. That is, the articular cartilage retains its cushioning properties by collagen and proteoglycan.

A reduction in supply of oxygen to the joint due to a lack of blood flow around the joint decreases the generation of, for example, proteoglycan by chondrocytes and causes stimulation by dead chondrocytes and inflammation of synovium, leading to occurrence of pain in the joint. The release of cytokines during inflammation further induces chondrocyte death, resulting in severity of symptoms of pain.

Accordingly, examples of the symptomatic treatment of OA include administration of a painkiller or an anti-inflammatory agent and injection of high-molecular hyaluronic acid (sodium hyaluronate) into the articular cavity. As treatment other than the symptomatic treatment, examples of the recent approach include enhancement of differentiation into chondrocytes, suppression of enlargement of chondrocytes, and enhancement of transcription and synthesis of collagen and proteoglycan by chondrocytes.

Proteoglycan is a structural component of cartilage and consists of sulfated glycosaminoglycan (GAG), which is a carbohydrate chain, and core protein covalently bonded to the GAG. In addition to the above-described effect of proteoglycan on OA, it is known that the amount of proteoglycan in a chondrocyte increases in the process of differentiation and proliferation from prechondrocyte to chondrocyte (Non Patent Document 1). It is thus suggested that proteoglycan has an important role in chondrogenesis. In addition, the proteoglycan contained in salmon cartilage has been reported to have effects as an anti-obesity drug or an antidiabetic drug (Patent Document 1) and to be useful for preventing and treating inflammatory disease or autoimmune disease, suppressing the rejection in organ transplantation, or treating allergy (Patent Document 2). Furthermore, an effect as an external preparation for anti-aging of the skin by promoting the in vivo synthesis of proteoglycan has been reported (Patent Document 3).

There has been reported on biosynthesis of proteoglycan: in cells highly expressing a transcription factor, Sox9, differentiation of chondrocytes is promoted and increased amounts of proteoglycan and type II collagen are generated (Non Patent Document 2); and in mice having mutated condroitin 4-O-sulfotransferase 1 (C4ST-1), which transfers a sulfate group to position 4 of GalNAc in condroitin 4-sulfate of sulfated glycosaminoglycan, the amount of synthesis of condroitin sulfate decreases and syndromes of achondroplasia are observed (Non Patent Document 3). Accordingly, it is believed that promotion of activities of Sox9 and C4ST-1 is important for biosynthesis of proteoglycan.

### RELATED ART

Patent Document 1: Japanese Patent Laid-Open No. 2010-126461
Patent Document 2: Japanese Patent Laid-Open No. 2007-131548
Patent Document 3: Japanese Patent Laid-Open No. 2006-028071

### NON-PATENT DOCUMENT

Non Patent Document 1: J. Biol. Chem., 265, 10, 5903-5909, 1990
Non Patent Document 2: Biochem. Biophys. Res. Commun., 301, 2, 338-343, 2003
Non Patent Document 3: BMC Musculoskelet Disord., 26, 2004

### SUMMARY OF INVENTION

It is an object of the present invention to provide a novel chondrogenesis promoter. Another object of the invention is to provide a novel proteoglycan synthesis promoter.

### SOLUTION TO PROBLEM

The present invention is composed of the following aspects:
(1) A chondrogenesis promoter containing a milk-derived basic protein fraction as an active ingredient;
(2) The chondrogenesis promoter according to aspect (1), wherein chondrogenesis is promoted by promoting the synthesis of proteoglycan;
(3) A proteoglycan synthesis promoter containing a milk-derived basic protein fraction as an active ingredient;
(4) A joint disease-preventing, ameliorating, or treating agent containing a milk-derived basic protein fraction as an active ingredient;
(5) The joint disease-preventing, ameliorating, or treating agent according to aspect (4), wherein the joint disease is degenerative joint disease;
(6) A joint disease-preventing or ameliorating supplement containing a milk-derived basic protein fraction as an active ingredient;
(7) The joint disease-preventing or ameliorating supplement according to aspect (6), wherein the joint disease is degenerative joint disease;
(8) Chondrogenesis-promoting food or drink and/or feed containing the chondrogenesis promoter according to aspect (1) or (2);
(9) Food or drink and/or feed containing the proteoglycan synthesis promoter according to aspect (3);
(10) A chondrogenesis promoter containing a decomposition product of a milk-derived basic protein fraction as an active ingredient;
(11) The chondrogenesis promoter according to aspect (10), wherein chondrogenesis is promoted by promoting the synthesis of proteoglycan;
(12) A proteoglycan synthesis promoter containing a decomposition product of a milk-derived basic protein fraction as an active ingredient;
(13) A joint disease-preventing, ameliorating, or treating agent containing a decomposition product of a milk-derived basic protein fraction as an active ingredient;
(14) The joint disease-preventing, ameliorating, or treating agent according to aspect (13), wherein the joint disease is degenerative joint disease;
(15) A joint disease-preventing or ameliorating supplement containing a decomposition product of a milk-derived basic protein fraction as an active ingredient;
(16) The joint disease-preventing or ameliorating supplement according to aspect (15), wherein the joint disease is degenerative joint disease;
(17) A chondrogenesis-promoting food or drink and/or feed containing the chondrogenesis promoter according to aspect (10) or (11) ;
(18) Food or drink and/or feed containing the proteoglycan synthesis promoter according to aspect (12);
(19) The chondrogenesis promoter according to aspect (10) or (11), wherein the decomposition product of the milk-derived basic protein fraction is prepared by treating a milk-derived basic protein fraction with a protease;
(20) The chondrogenesis promoter according to aspect (19), wherein the protease is at least one selected from the group consisting of pepsin, trypsin, chymotrypsin, pancreatin, and papain; and
(21) A method of preventing, ameliorating, or treating joint disease, the method including:
   ingestion of not less than 10 mg/day of a milk-derived basic protein fraction and/or a decomposition product of the milk-derived basic protein fraction.

### EFFECTS OF INVENTION

The chondrogenesis promoter of the present invention promotes differentiation of chondrocytes and synthesis of proteoglycan by promoting expression of mRNAs of Sox9 and condroitin 4-O-sulfotransferase 1 (C4ST-1), which are chondrocyte differentiation regulators, and can effectively promote chondrogenesis. The proteoglycan synthesis promoter of the present invention also promotes the synthesis of proteoglycan and has a joint disease-preventing, ameliorating, or treating effect.

### BRIEF DESCRIPTION OF DRAWINGS

Fig. 1 shows concentration dependence of a milk-derived basic protein fraction on the expression of C4ST-1 mRNA.
Fig. 2 shows time dependence of a milk-derived basic protein fraction on the expression of C4ST-1 mRNA.
Fig. 3 shows concentration dependence of a milk-derived basic protein fraction on the expression of cartilage differentiation regulator, Sox9, mRNA.
Fig. 4 shows time dependence of a milk-derived basic protein fraction on the expression of cartilage differentiation regulator, Sox9, mRNA.
Fig. 5 shows influence of a milk-derived basic protein fraction on the synthetic quantity of proteoglycan.
Fig. 6 shows the effect of a milk-derived basic protein fraction on knee osteoarthritis.
Fig. 7 shows the influence of a decomposition product of a milk-derived basic protein fraction on the synthetic quantity of proteoglycan.
Fig. 8 shows the effect of a decomposition product of a milk-derived basic protein fraction on knee osteoarthritis.

### DESCRIPTION OF EMBODIMENT

The present invention is characterized in that a milk-derived basic protein fraction is used as an active ingredient.

The milk-derived basic protein fraction of the present invention has the following characteristics:
1) the fraction is composed of several proteins having molecular weights in a range of 3000 to 80000 by sodium dodecyl sulfate-polyacrylamide gel electrophoresis (SDS-PAGE);
2) the fraction contains 95 wt% or more of the proteins and small amounts of fats and ash;
3) the proteins are mainly composed of lactoferrin and lactoperoxidase; and
4) the proteins each have an amino acid composition containing 15 wt% or more of basic amino acids such as lysine, histidine, and arginine.

The milk-derived basic protein fraction can be prepared from mammal milks such as cow milk, human milk, goat milk, and sheep milk by any known method, for example, a method involving bringing milk or a milk-derived raw material into contact with a cation exchanger to allow basic proteins to adsorb thereon, and eluting the basic protein fraction adsorbed to the cation exchanger with an elute having a pH of higher than 5 and an ionic strength of higher than 0.5 (Japanese Patent Laid-Open No. H05-202098), a method using alginate gel (Japanese Patent Laid-Open No. S61-246198), a method of preparing a fraction from whey with inorganic porous particles (Japanese Patent Laid-Open No. H01-086839), or a method of preparing a fraction from milk with a sulfated ester compound (Japanese Patent Laid-Open No. S63-255300). The present invention can use a milk-derived basic protein fraction prepared by such a method. The prepared milk-derived basic protein fraction may be further treated with a protease, and the resulting decomposition product of the milk-derived basic protein fraction having an average molecular weight of 4000 or less may be used. Usable examples of the protease include commercially available proteases for food or industrial uses, such as Protease A "Amano" SD (trade name), THERMOASE PC10F (trade name), and PROTIN SD-AY10 (trade name); and enzymes such as pepsin, trypsin, chymotrypsin, pancreatin, and papain. These proteases may be used in an appropriate combination.

In the present invention, the milk-derived basic protein fraction can be directly used as a chondrogenesis promoter or a proteoglycan synthesis promoter. Alternatively, the milk-derived basic protein fraction may be mixed with other materials that are usually used in drugs, food or drink, or feed, such as saccharides, fats, proteins, vitamins, minerals, or flavors, and may be further formulated into, for example, powder, granules, tablets, capsules, or drinkable preparations. The fraction may also be used in liniments in the form of usual application formulations, such as emulsion, cream, lotion, or packs. These liniments can be produced through a common method involving appropriately compounding the milk-derived basic protein fraction of the present invention as an active ingredient in the production process.

In addition, another component having a chondrogenesis-promoting effect or another component having a joint disease-ameliorating effect may be used together with the milk-derived basic protein fraction.

The chondrogenesis promoter of the present invention may be compounded in any amount that an adult can ingest 10 mg/day or more of the milk-derived basic protein fraction. Such an amount is expected to exhibit a chondrogenesis-promoting effect or a proteoglycan synthesis-promoting effect.

The milk-derived chondrogenesis-promoting food or drink and proteoglycan synthesis-promoting food or drink of the present invention can be prepared by compounding the milk-derived basic protein fraction to ordinary food or drink such as yogurt, milk beverage, wafer, or desert. The amount of the basic protein fraction contained in chondrogenesis-promoting food or drink, which varies depending on the formulation of the food or drink, and preferably ranges 1 to 100 mg for 100 g of the food or drink, for ensuring ingestion of 10 mg/day or more of the milk-derived basic protein fraction by an adult. The chondrogenesis promoter of the present invention may be prepared by mixing the milk-derived basic protein fraction with ordinary feed, such as feed for livestock or pet food. For example, the milk-derived basic protein fraction is compounded to such feed in an amount of preferably 1 to 100 mg for 100 g of the feed.

In the present invention, the milk-derived basic protein fraction may be compounded by any process. For example, in a case of adding or compounding a solution of the milk-derived basic protein fraction, the fraction is dispersed or dissolved in deionized water with stirring and is then formulated into a drug, food or drink, or feed. The stirring may be performed under any condition for homogenous mixing of the milk-derived basic protein fraction. For example, an ultra-disperser or TK homomixer may be used. The solution of the composition may be concentrated with an RO membrane for example or lyophilized as necessary to be readily formulated into a drug, food or drink, or feed. In the present invention, sterilization treatment that is usually employed in production of a drug, food or drink, or feed can be performed. In a powder form, dry heat sterilization can also be employed. Accordingly, a drug, food or drink, or feed containing the milk-derived basic protein fraction of the present invention can be produced in various formulations, such as a liquid, gel, powder, or granule form.

The present invention will now be described in more detail by examples and test examples, which are merely exemplification, and the invention should not be limited to these examples.

### [Example 1]

A column (diameter: 5 cm, height: 30 cm) filled with 400 g of sulfonated Chitopearl (a cation exchange resin, manufactured by Fuji Spinning Co., Ltd.) was thoroughly washed with deionized water, and 40 L of unsterilized skim milk (pH 6.7) was supplied to the column at a flow rate of 25 mL/min. The column was then thoroughly washed with deionized water, and the basic protein fraction adsorbed onto the resin was eluted with 0.02 M carbonate buffer (pH 7.0) containing 0.98 M sodium chloride. The eluate was desalinated with a reverse osmosis membrane (RO membrane), was concentrated, and was then lyophilized to give 21 g of a milk-derived basic protein fraction powder (Example Product 1). The resulting milk-derived basic protein fraction was subjected to sodium dodecyl sulfate-polyacrylamide gel electrophoresis (SDS-PAGE) to confirm that the molecular weight distributed in the range of 3000 to 80000 and that the composition was as shown in Table 1. The fraction was hydrolyzed with 6 N hydrochloric acid at 110°C for 24 hours and was then subjected to amino acid composition analysis with an amino acid analyzer (model: L-8500, manufactured by Hitachi, Ltd.). As shown in Table 2, the results demonstrated that the amount of basic amino acids was 15 wt% or more. The protein composition was further analyzed by ELISA. As shown in Table 3, the amounts of lactoferrin and lactoperoxidase were each 40% or more.

**[Table 1]**

| Results of component analysis | |
|---|---|
| Water | 1.06(wt%) |
| Protein | 96.50 |
| Lipid | 0.56 |
| Ash | 0.27 |
| Others | 1.61 |

**[Table 2]**

| Results of amino acid analysis | |
|---|---|
| Aspartic acid | 10.1(wt%) |
| Serine | 5.3 |
| Glutamic acid | 12.3 |
| Proline | 4.7 |
| Alanine | 5.7 |
| Leucine | 10.2 |
| Lysine | 8.4 |
| Histidine | 2.5 |
| Arginine | 7.2 |
| Others | 33.6 |

**[Table 3]**

| Results of protein composition analysis | |
|---|---|
| Lactoferrin | 42.5(wt%) |
| Lactoperoxidase | 45.6 |
| Insulin-like growth factor | 0.005 |
| Others | 11.895 |

### [Test Example 1]

The influence of a milk-derived basic protein fraction on the expression of proteoglycan synthesis enzyme mRNA was examined by real time PCR using Example Product 1 as the milk-derived basic protein fraction and using mouse EC-derived mesenchymal cells, ATDC5 cells. ATDC5 cells were seeded in a 24-well plate at 0.5 × 10⁵ cells/well and were cultured in a DMEM/F12 medium (manufactured by Sigma) at 37°C in a 5% CO₂ environment for 1 week.

The milk-derived basic protein fraction and purified lactoferrin were each dissolved in a DMEM/F12 medium into concentrations of 0.1%, 0.5%, and 1% (w/v). The lactoferrin solutions were used as a control group. Each solution was added to the cultured cells, followed by culturing for 12 hours.

Total RNA was collected from the cultured cells, and cDNA was synthesized. In detail, 0.5 mL of an RNA extracting agent, ISOGEN (manufactured by Nippon Gene Co., Ltd.), was added to the cultured cells, followed by leaving to stand for 5 minutes. The cells were solubilized by pipetting, and the cell solution was collected in a 1.5-mL tube. To the cell solution was added 0.1 mL of chloroform. The mixture was sufficiently stirred. After separation into two layers, the upper layer (aqueous layer) was collected in another 1.5-mL tube. To the collected solution was added 0.25 mL of 2-propyl alcohol. The mixture was left to stand for 10 minutes and was then centrifuged at 15000 rpm at 4°C for 15 minutes to give a precipitate of total RNA. The resulting precipitate was washed with 70% ethanol and was dissolved in DEPC water to give an RNA solution. Complementary DNA was synthesized from 1 µg of the RNA with Takara PrimeScript^{™} RT reagent Kit.

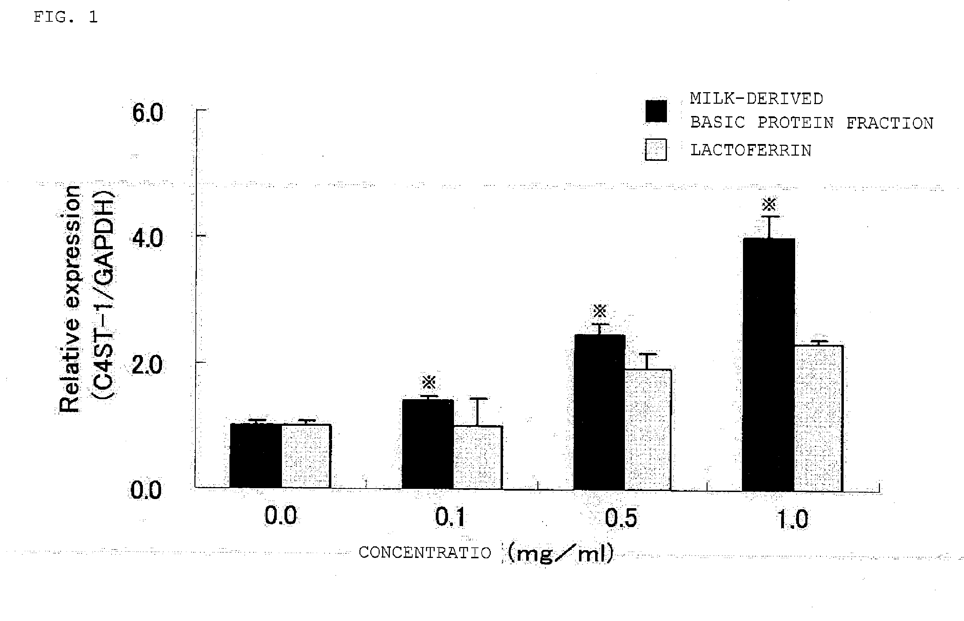

Real time PCR using the resulting cDNA as a template was performed with SYBR Green (Takara SYBR Prime Ex Taq II) under the reaction conditions: initial denaturation at 95°C for 30 seconds followed by 40 cycles of denaturation at 95°C for 5 seconds, annealing at 57°C for 15 seconds, and extension at 72°C for 20 seconds. The primers used were those for C4ST1, shown in Table 4. The results are shown in Fig. 1. The symbol * indicates a significant difference compared to the control group (p < 0.05).

**[Table 4]**

| | | |
|---|---|---|
| **C4ST1** | **forward** primer | 5'-TCCACAAGCGCTACGGCACCAAGATC-3' |
| | **reverse** primer | 5'-AGGGCCTCCTGCGTGGCGTTCTTCC-3' |

Fig. 1 reveals that the expression of C4ST-1 mRNA is increased depending on the concentration of the milk-derived basic protein fraction. In addition, it was revealed that the milk-derived basic protein fraction exhibits a higher effect of enhancing the expression of C4ST-1 mRNA, compared to a milk protein, lactoferrin.

### [Test Example 2]

The influence of a milk-derived basic protein fraction on the expression of proteoglycan synthesis enzyme mRNA was examined by real time PCR changing the treatment time and using Example Product 1 as the milk-derived basic protein fraction.

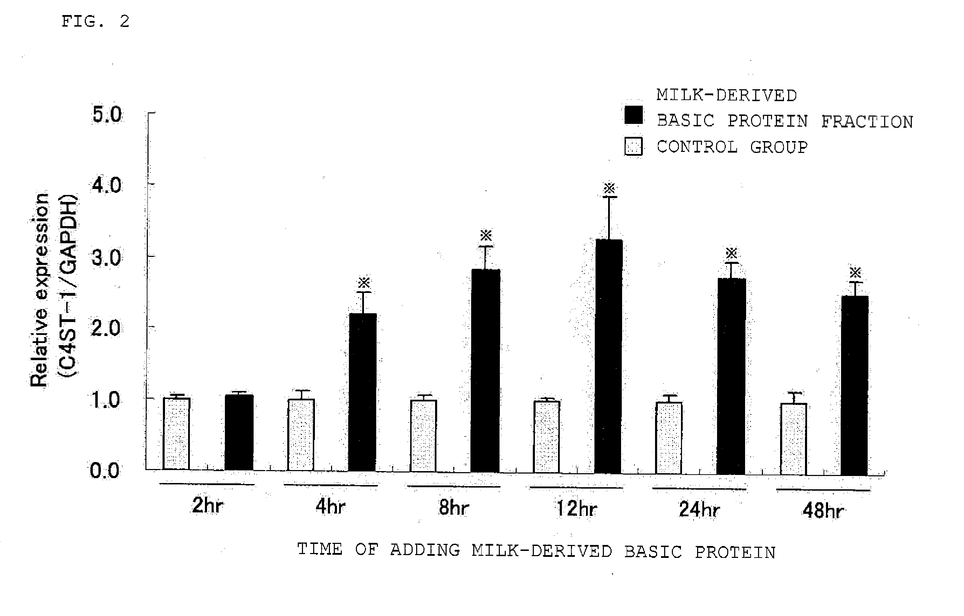

The method was performed as in the method described in Test Example 1. In detail, the milk-derived basic protein fraction was added to ATDC5 cells for 2 hours to for 48 hours. Total RNA was collected, and cDNA was synthesized, followed by real time PCR. In the control group, total RNA was collected from the ATDC5 cells cultured for 2 hours to for 48 hours without administering the milk-derived basic protein fraction, and cDNA was synthesized, followed by real time PCR. The results are shown in Fig. 2.

Fig. 2 shows that the expression of C4ST-1 mRNA was significantly enhanced by the milk-derived basic protein fraction at 4 hours after the action of the milk-derived basic protein fraction on ATDC cells, and the action reached the maximum at 12 hours to 24 hours and turned to a decrease at 48 hours.

### [Test Example 3]

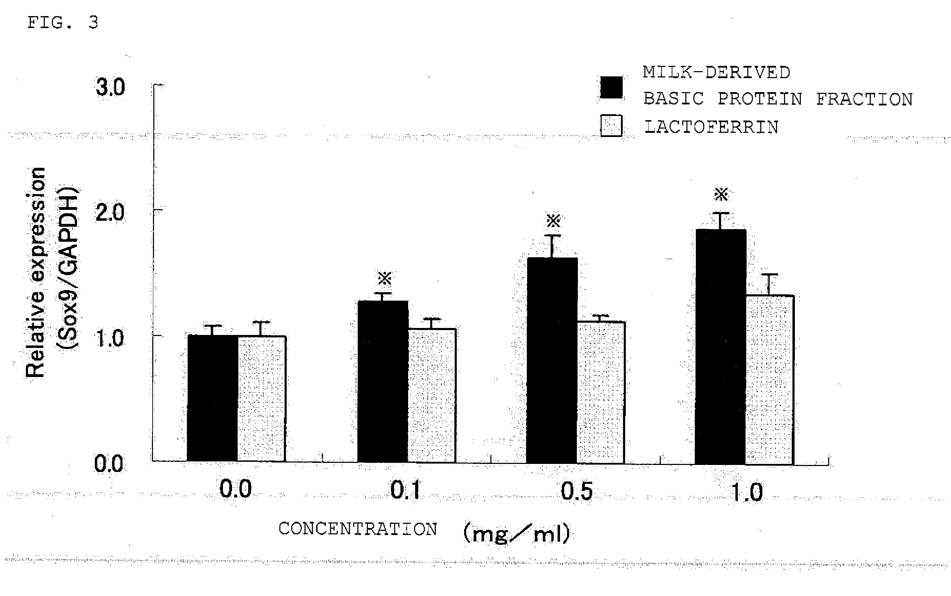

The influence of a milk-derived basic protein fraction on the expression of cartilage differentiation regulator mRNA was examined by real time PCR using Example Product 1 as the milk-derived basic protein fraction. As in Test Example 1, purified lactoferrin was used as a control group. The method was performed as in the method described in Test Example 1. The primers used were those for Sox9 shown in Table 5. The results are shown in Fig. 3.

**[Table 5]**

| | | |
|---|---|---|
| **SOX9** | **forward** primer | 5'-GAGGCCACGGAACAGACTCA-3' |
| | **reverse** primer | 5'-CAGCGCCTTGAAGATAGCATT-3' |

The results shown in Fig. 3 reveal that the expression of Sox9 mRNA by the milk-derived basic protein fraction is enhanced depending on the concentration of whey decomposition product. In addition, it was also revealed that the milk-derived basic protein fraction exhibits a higher effect of enhancing the expression of Sox9 mRNA, compared to a milk protein, lactoferrin.

### [Test Example 4]

The influence of a milk-derived basic protein fraction on the express of cartilage differentiation regulator mRNA was examined by real time PCR changing the treatment time and using Example Product 1 as the milk-derived basic protein fraction.

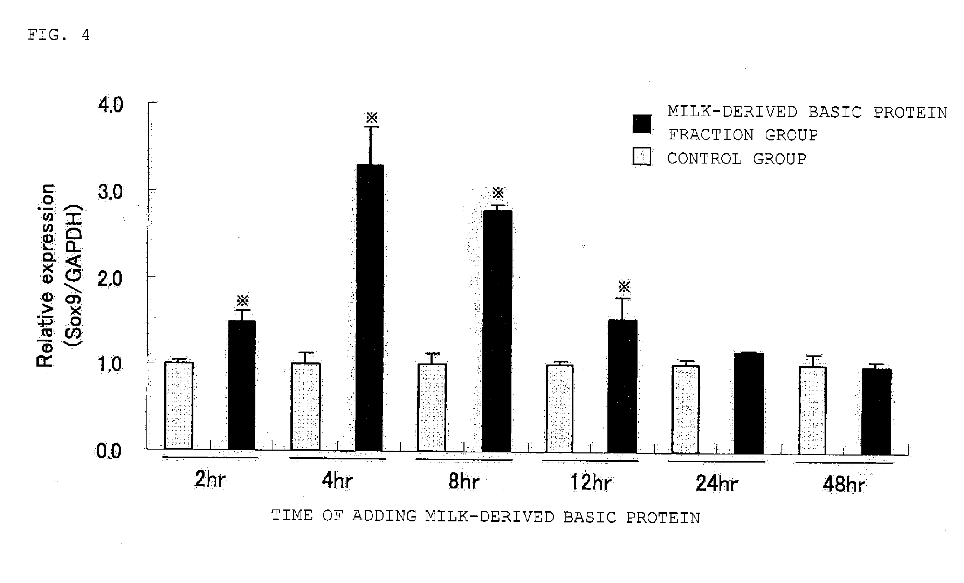

The method was performed as in the method described in Test Example 1. In detail, the milk-derived basic protein fraction was added to ATDC5 cells for 2 hours to for 48 hours. Total RNA was collected, and cDNA was synthesized, followed by real time PCR. In the control group, total RNA was collected from the ATDC5 cells cultured for 2 hours to for 48 hours without administering the milk-derived basic protein fraction, and cDNA was synthesized, followed by real time PCR. The results are shown in Fig. 4.

Fig. 4 shows that the expression of Sox9 mRNA by the milk-derived basic protein fraction was significantly increased at 2 hours after the action of the milk-derived basic protein fraction on ATDC cells, and the action reached the maximum at 4 hours to 6 hours and turned to a decrease at 12 hours.

### [Test Example 5]

The influence of a milk-derived basic protein fraction on proteoglycan synthesis was investigated by measuring the amount of proteoglycan (the amount of condroitin sulfate) with an acid mucopolysaccharide measuring kit (AK03, manufactured by Primary Cell) using Example Product 1 as the milk-derived basic protein fraction. The measurement was performed as follows.

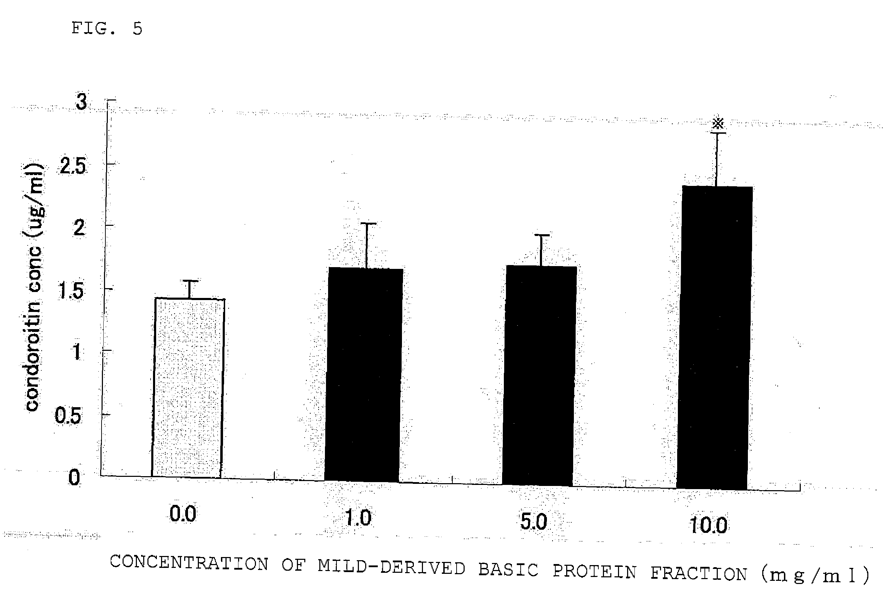

ATDC5 cells were seeded in a 12-well plate at 1 × 10⁵ cells/well and were cultured in a DMEM/F12 medium at 37°C in a CO₂ environment. On the fourth day of the culture, the enzyme solution (one bag/10 mL water) of the kit was added to each well at 200 µL/well to solubilize the cells. The solubilization solution was collected in a 1.5-mL tube and was treated at 60°C for 1 hour to give a sample. The sample and a condroitin sulfate solution standard (1 to 50 µg/mL), each 100 µL, were dispensed in a tube, and 1.3 mL of a reaction solution (0.4 mL of undiluted color developing solution and 12.6 mL of buffer) was added thereto. The absorbance or OD was measured at 650 nm within 10 to 20 minutes after the addition. The concentration of condroitin was determined based on the standard curve determined from the absorption of the condroitin sulfate solution standards and the absorbance of the sample. The results are shown in Fig. 5.

Fig. 5 shows that the amount of proteoglycan was significantly increased depending on the concentration of the milk-derived basic protein fraction.

### [Test Example 6]

The influence of a milk-derived basic protein fraction on chondrogenesis was investigated by administering Example Product 1 as the milk-derived basic protein fraction to spontaneous knee osteoarthritic mice, STR/ORT mice. The measurement was performed by the following process. Ten 15-week old STR/ORT mice were administered with 10 mg of the milk-derived basic protein fraction per kg body weight, and another ten 15-week old STR/ORT mice were administered with physiological saline. The administration was performed once a day by forced oral administration with a mouse-feeding metal stomach tube. In addition, 10 normal mice (CBA/JN) were bred as a normal control group. After breeding for 12 weeks, the mice of each group were slaughtered to obtain the hind-limb knee joints. The bone and cartilage tissue was washed with a phosphate buffered saline solution (PBS) of 4°C, was fixed with 4% paraformaldehyde in PBS at 4°C for 18 hours, and was further delipidated overnight with a series of 70% to 100% ethanol solutions. The tissue was immersed in a phosphate buffer containing 10% ethylene diamine tetraacetate (EDTA) for decalcification at 4°C for 2 weeks. The tissue was embedded in paraffin and was cut into thin specimens of 4 µm each. The tissue specimens were deparaffinized and hydrophilized again and were then stained with Safranin-O. The degrees of progress of arthritis were scored in accordance with the Mankin score system (Table 6).

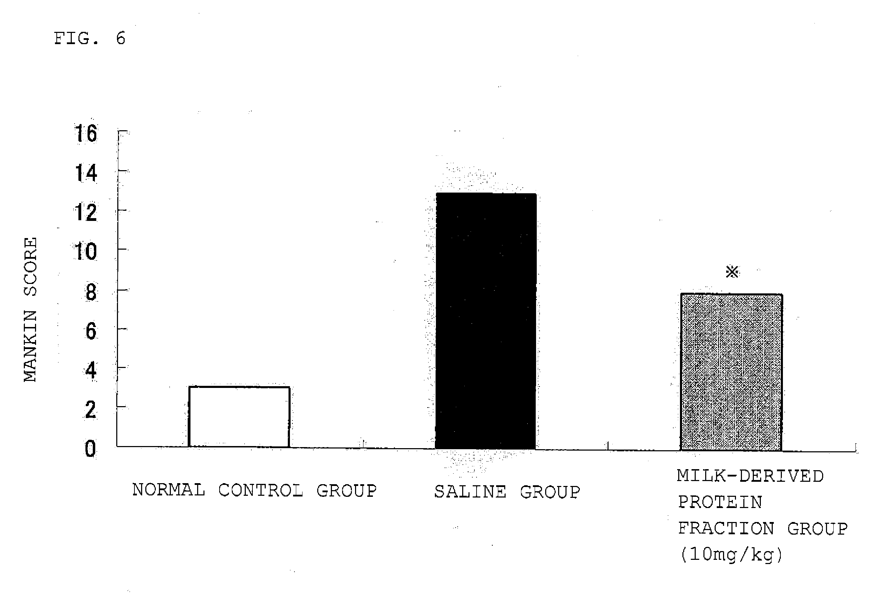

The results are shown in Fig. 6.

**[Table 6]**

| Mankin Score System | |
|---|---|
| | Score |
| I Structure | |
| a. Normal | 0 |
| b. Irregular surface | 1 |
| c. Irregular surface and pannus formation | 2 |
| d. Fissure reaching the intermediate layer | 3 |
| e. Fissure reaching the deep layer | 4 |
| f. Fissure reaching the calcification layer | 5 |
| g. Complete destruction | 6 |
| II Cell | |
| a. Normal | 0 |
| b. Increased number of diffuse cells | 1 |
| c. Cloning | 2 |
| d. Decreased number of cells | 3 |
| III Safranin-O staining | |
| a. Normal | 0 |
| b. Slight decrease | 1 |
| c. Moderate decrease | 2 |
| d. Large decrease | 3 |
| e. Disappearance of staining | 4 |
| IV Tidemark condition | |
| a. Normal | 0 |
| b. Crossing of blood vessels | 1 |
| Total score | 0 to 14 |

Fig. 6 demonstrates that the Mankin scores of STR/ORT mice were significantly decreased by administration of the milk-derived basic protein fraction, compared to that by administration of saline administration. This demonstrates that the administration of the milk-derived basic protein fraction promotes the chondrogenesis of mice to relieve the symptoms of knee osteoarthritis.

### [Example 2]

### (Preparation of liquid nutrition composition)

Five grams of Example Product 1 as a milk-derived basic protein fraction was dissolved in 4995 g of deionized water. The solution was stirred with a TK homomixer (TKROBO MICS, manufactured by Tokusyu Kika Kogyo Co., Ltd.) at 6000 rpm for 30 minutes to give a milk-derived basic protein fraction solution containing 100 mg of the milk-derived basic protein fraction for 100 g of the solution. To 5.0 kg of this milk-derived basic protein fraction solution were added 4.0 kg of casein, 5.0 kg of soybean protein, 1.0 kg of fish oil, 3.0 kg of perilla oil, 18.0 kg of dextran, 6.0 kg of a mineral mixture, 1.95 kg of vitamin mixture, 2.0 kg of an emulsifier, 4.0 kg of a stabilizer, and 0.05 kg of a flavor. The mixture was packed in a 200-mL retort pouch and was sterilized with a retort sterilizer (class-1 pressure vessel, type: RCS-4CRTGN, manufactured by Hisaka Works, Ltd.) at 121°C for 20 minutes to produce 50 kg of a liquid nutrition composition of the present invention. In the resulting liquid nutrition composition, no precipitate was observed, and no abnormal flavor was sensed. This liquid nutrition composition (100 g) contained 10 mg of the milk-derived basic protein fraction and had chondrogenesis-promoting and/or proteoglycan synthesis-promoting effect.

### [Example 3]

### (Preparation of gel-form food)

Two grams of Example Product 1 as a milk-derived basic protein fraction was dissolved in 708 g of deionized water. The mixture was stirred with an ultra-disperser (ULTRA-TURRAXT-25, manufactured by IKA Japan K.K.) at 9500 rpm for 30 minutes. To the resulting solution were added 40 g of sorbitol, 2 g of an acidifier, 2 g of a flavor, 5 g of pectin, 5 g of a whey protein concentrate, 1 g of calcium lactate, and 235 g of deionized water. The mixture was stirred and mixed and was then charged in a 200-mL cheer pack and was sterilized at 85°C for 20 minutes, followed by hermetic sealing to give 5 bags (each content: 200 g) of gel-form food of the present invention. In the resulting gel-form food, no precipitate was observed, and no abnormal flavor was sensed. This gel-form food (100 g) contained 200 mg of the milk-derived basic protein fraction and had chondrogenesis-promoting and/or proteoglycan synthesis-promoting effect.

### [Example 4]

### (Preparation of drink)

Two grams of an acidifier was dissolved in 706 g of deionized water, and 4 g of Example Product 1 as a milk-derived basic protein fraction was dissolved therein. The mixture was stirred with an ultra-disperser (ULTRA-TURRAXT-25, manufactured by IKA Japan K.K.) at 9500 rpm for 30 minutes. To the resulting solution were added 100 g of maltitol, 20 g of reduced sugar syrup, 2 g of a flavor, and 166 g of deionized water. The mixture was charged in a 100-mL glass bottle and was sterilized at 95°C for 15 seconds, followed by hermetic sealing to give 10 bottles (each content: 100 mL) of drink. In the resulting drink, no precipitate was observed, and no abnormal flavor was sensed. This drink (100 g) contained 400 mg of the milk-derived basic protein fraction and had chondrogenesis-promoting and/or proteoglycan synthesis-promoting effect.

### [Example 5]

### (Preparation of feed)

Two kilograms of Example Product 1 as a milk-derived basic protein fraction was dissolved in 98 kg of deionized water. The solution was stirred with a TK homomixer (model: MARKII 160, manufactured by Tokusyu Kika Kogyo Co., Ltd.) at 3600 rpm for 40 minutes to give a milk-derived basic protein fraction solution containing 2 g of the milk-derived basic protein fraction for 100 g of the solution. To 10 kg of this milk-derived basic protein fraction solution were added 12 kg of soybean cake, 14 kg of skim milk, 4 kg of soybean oil, 2 kg of corn oil, 23.2 kg of palm oil, 14 kg of corn starch, 9 kg of flour, 2 kg of wheat bran, 5 kg of a vitamin mixture, 2.8 kg of cellulose, and 2 kg of a mineral mixture. The mixture was sterilized at 120°C for 4 minutes to give 100 kg of dog breeding feed of the present invention. This dog breeding feed (100 g) contained 200 mg of the milk-derived basic protein fraction and had chondrogenesis-promoting and/or proteoglycan synthesis-promoting effect.

### [Example 6]

### (Preparation of tablet)

Raw materials were mixed at formulations shown in Table 4 and were formed into tablets each having a weight of 1 g of the present invention by a common method. This tablet (1 g) contained 100 mg of the milk-derived basic protein fraction and had chondrogenesis-promoting and/or proteoglycan synthesis-promoting effect.

**[Table 7]**

| | |
|---|---|
| Crystalline glucose hydrate | 83.5% (wt%) |
| Milk-derived basic protein fraction (Example Product 1) | 10.0% |
| Mineral mixture | 5.0% |
| Sugar ester | 1.0% |
| Flavor | 0.5% |

### [Example 7]

A milk-derived basic protein fraction powder was prepared as in Example 1, and 50 g of the powder was dissolved in 10 L of distilled water. The solution was then reacted with 1% pancreatin (manufactured by Sigma) at 37°C for 2 hours. The reaction solution was treated with heat of 80°C for 10 minutes to deactivate the enzyme. Consequently, 48.3 g of a decomposition product (Example Product 7) of the milk-derived basic protein fraction was prepared.

### [Example 8]

A milk-derived basic protein fraction powder was prepared as in Example 1, and 120 g of the powder was dissolved in 1.8 L of purified water. The solution was maintained at 45°C and was reacted with 20 g of Protease A "Amano" SD (manufactured by Amano Enzyme Inc.) at the temperature for 2 hours. The reaction solution was treated with heat of 80°C for 10 minutes to deactivate the enzyme. Consequently, 95 g of a decomposition product (Example Product 8) of the milk-derived basic protein fraction was prepared.

### [Test Example 7]

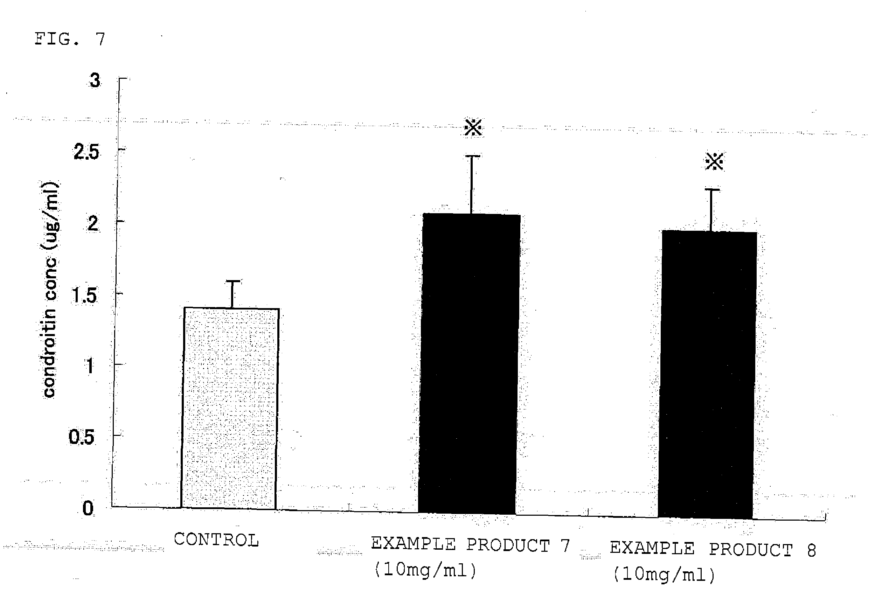

The influence of a decomposition product of a milk-derived basic protein fraction on proteoglycan synthesis was inspected using Example Products 7 and 8. The inspection was conducted as in Test Example 5. The results are shown in Fig. 7.

Fig. 7 demonstrates that the decomposition products of the milk-derived basic protein fractions significantly increase the amount of proteoglycan.

### [Test Example 8]

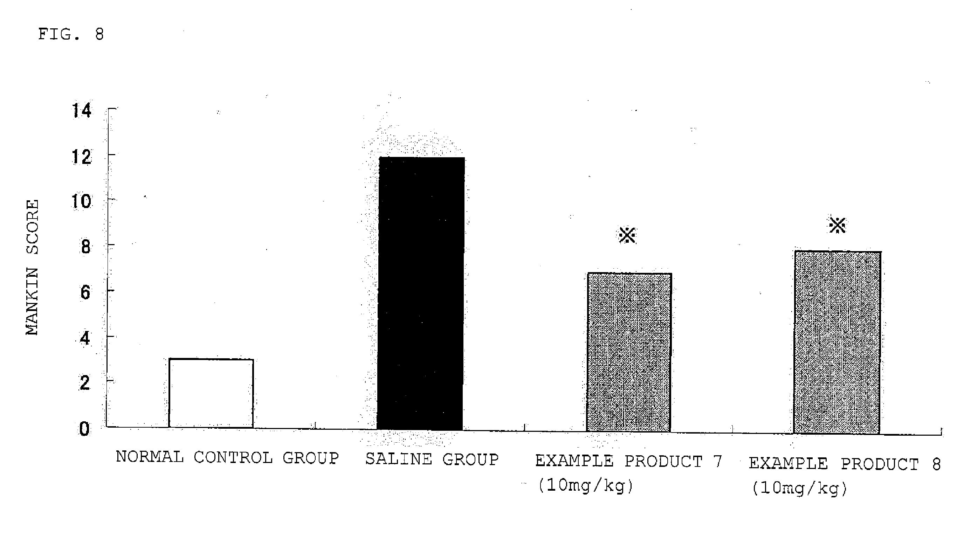

The influence of a decomposition product of a milk-derived basic protein fraction on chondrogenesis was inspected using Example Products 7 and 8. The inspection was conducted as in Test Example 6 by administering the decomposition product of the milk-derived basic protein fraction to each STR/ORT mouse in an amount of 10 mg for 1 kg of body weight. The results are shown in Fig. 8.

The results shown in Fig. 8 demonstrate that administration of the decomposition product of the milk-derived basic protein fraction significantly decreases the Mankin scores of STR/ORT mice compared to administration of physiological saline, like the milk-derived basic protein fraction. That is, administration of a decomposition product of a milk-derived basic protein fraction promotes the chondrogenesis of mice to relieve the symptoms of knee osteoarthritis.

### (Preparation of drink)

Two grams of an acidifier was dissolved in 706 g of deionized water, and 4 g of Example Product 7 as the decomposition product of a milk-derived basic protein fraction was dissolved therein. The mixture was stirred with an ultra-disperser (ULTRA-TURRAXT-25, manufactured by IKA Japan K.K.) at 9500 rpm for 30 minutes. To the resulting solution were added 100 g of maltitol, 20 g of reduced sugar syrup, 2 g of a flavor, and 166 g of deionized water. The mixture was charged in a 100-mL glass bottle and was sterilized at 95°C for 15 seconds, followed by hermetic sealing to give 10 bottles (each content: 100 mL) of drink. In the resulting drink, no precipitate was observed, and no abnormal flavor was sensed. This drink (100 g) contained 400 mg of the decomposition product of the milk-derived basic protein fraction and had chondrogenesis-promoting and/or proteoglycan synthesis-promoting effect.

### [Example 9]

### (Preparation of tablet)

Raw materials were mixed at formulations shown in Table 8 and were formed into tablets each having a weight of 1 g of the present invention by a common method. This tablet (1 g) contained 100 mg of the decomposition product of the milk-derived basic protein fraction and had chondrogenesis-promoting and/or proteoglycan synthesis-promoting effect.

### [Example 10]

**[Table 8]**

| | |
|---|---|
| Crystalline glucose hydrate | 83.5% (wt%) |
| Milk-derived basic protein fraction decomposition product (Example Product 8) | 10.0% |
| Mineral mixture | 5.0% |
| Sugar ester | 1.0% |
| Flavor | 0.5% |

## Claims

1. A chondrogenesis promoter comprising:
a milk-derived basic protein fraction as an active ingredient.

2. The chondrogenesis promoter according to Claim 1, wherein
chondrogenesis is promoted by promoting the synthesis of proteoglycan.

3. A proteoglycan synthesis promoter comprising:
a milk-derived basic protein fraction as an active ingredient.

4. A joint disease-preventing, ameliorating, or treating agent comprising:
a milk-derived basic protein fraction as an active ingredient.

5. The joint disease-preventing, ameliorating, or treating agent according to Claim 4, wherein
the joint disease is degenerative joint disease.

6. A joint disease-preventing or ameliorating supplement comprising:
a milk-derived basic protein fraction as an active ingredient.

7. The joint disease-preventing or ameliorating supplement according to Claim 6, wherein
the joint disease is degenerative joint disease.

8. Chondrogenesis-promoting food or drink and/or feed comprising:
the chondrogenesis promoter according to Claim 1 or 2.

9. Food or drink and/or feed comprising:
the proteoglycan synthesis promoter according to Claim 3.

10. A chondrogenesis promoter comprising:
a decomposition product of a milk-derived basic protein fraction as an active ingredient.

11. The chondrogenesis promoter according to Claim 10, wherein
chondrogenesis is promoted by promoting the synthesis of proteoglycan.

12. A proteoglycan synthesis promoter comprising:
a decomposition product of a milk-derived basic protein fraction as an active ingredient.

13. A joint disease-preventing, ameliorating, or treating agent comprising:
a decomposition product of a milk-derived basic protein fraction as an active ingredient.

14. The joint disease-preventing, ameliorating, or treating agent according to Claim 13, wherein
the joint disease is degenerative joint disease.

15. A joint disease-preventing or ameliorating supplement comprising:
a decomposition product of a milk-derived basic protein fraction as an active ingredient.

16. The joint disease-preventing or ameliorating supplement according to Claim 15, wherein
the joint disease is degenerative joint disease.

17. A chondrogenesis-promoting food or drink and/or feed comprising:
the chondrogenesis promoter according to Claim 10 or 11.

18. Food or drink and/or feed comprising:
the proteoglycan synthesis promoter according to Claim 12.

19. The chondrogenesis promoter according to Claim 10 or 11, wherein
the decomposition product of the milk-derived basic protein fraction is prepared by treating a milk-derived basic protein fraction with a protease.

20. The chondrogenesis promoter according to Claim 19, wherein
the protease is at least one selected from the group consisting of pepsin, trypsin, chymotrypsin, pancreatin, and papain.

21. A method of preventing, ameliorating, or treating joint disease, the method comprising:
ingestion of not less than 10 mg/day of a milk-derived basic protein fraction and/or a decomposition product of the milk-derived basic protein fraction.
